Europäisches Patentamt

European Patent Office

Office européen des brevets

(19)

(11) Veröffentlichungsnummer: **0 257 511**
**B1**

(12) # EUROPÄISCHE PATENTSCHRIFT

(45) Veröffentlichungstag der Patentschrift:
07.11.90

(51) Int. Cl.⁵: **C07C 29/04,** C07C 31/10,
C07C 31/12, C07C 31/02

(21) Anmeldenummer: 87111908.7

(22) Anmeldetag: 17.08.87

(54) Verfahren zur Herstellung von Isopropylakohol und tertiären C4-bis C5-Alkoholen.

(30) Priorität: 19.08.86 DE 3628008
19.08.86 DE 3628007

(43) Veröffentlichungstag der Anmeldung:
02.03.88 Patentblatt 88/9

(45) Bekanntmachung des Hinweises auf die Patenterteilung:
07.11.90 Patentblatt 90/45

(84) Benannte Vertragsstaaten:
AT BE CH DE ES FR GB GR IT LI NL SE

(56) Entgegenhaltungen:
DE-A- 3 025 262
DE-B- 2 147 739
DE-B- 2 429 770
DE-C- 3 419 392
US-A- 4 352 945
US-A- 4 405 822
US-A- 4 469 905

(73) Patentinhaber: RWE-DEA Aktiengesellschaft für
Mineraloel und Chemie, Überseering 40,
D-2000 Hamburg 60(DE)

(72) Erfinder: Carls, Rolf-Rainer, Schillerstrasse 127,
D-4100 Duisburg 17(DE)
Erfinder: Dettmer, Michael, Auestrasse 17,
D-6057 Dietzenbach(DE)
Erfinder: Osterburg, Günther, Ahornstrasse 5,
D-4137 Rheurdt 2(DE)
Erfinder: Prezelj, Milan, Franz-Lenbachstrasse 10,
D-6000 Frankfurt/Main 70(DE)
Erfinder: Webers, Werner, Rektor-Horn-Strasse 42,
D-4134 Rheinberg 3(DE)

(74) Vertreter: Schupfner, Gerhard D., Müller, Schupfner &
Gauger Karlstrasse 5 Postfach 14 27,
D-2110 Buchholz/Nordheide(DE)

**Beschreibung**

Die vorliegende Erfindung betrifft ein Verfahren zur Herstellung von Isopropylalkohol und tertiären $C_4$ bis $C_5$-Alkoholen durch Umsetzung eines Kohlenwasserstoffstroms, der Propen bzw. $C_4$ bis $C_5$-Iso-olefine enthält, mit Wasser in Gegenwart eines starksauren Feststoff-Katalysators bei erhöhtem Druck und erhöhter Temperatur, Abführung des Restgases und des wäßrigen Produktalkohols und Gewinnung des Alkohols.

Durch die DE-AS 1 210 768 ist bereits ein Verfahren zur kontinuierlichen Herstellung von Isopropylalkohol und Diisopropyläther durch katalytische Hydratisierung von Propen bekannt geworden. Hierbei wird als Katalysator ein starksaurer Kationenaustauscher verwendet, der aus einem mit etwa 5 bis 20 Gewichtsprozent Divinylbenzol vernetztem Styrolpolymerisat, das pro aromatischem Ring etwa eine Sulfonsäuregruppe enthält, besteht. Bei diesem bekannten Verfahren wird zur Herstellung des Alkohols als Hauptprodukt unter einem Druck von etwa 17 bis 105 atm. bei einer Temperatur von etwa 135 bis 157°C und mit einem Verhältnis von 4 bis 10 Mol Wasser pro Mol Propen gearbeitet. Es sind ferner Beschickungsgeschwindigkeiten von 0,5 bis 10 Raumteilen flüssiges Propen pro Raumteil des feuchten Harzkatalysators und Stunde vorgesehen. Da die Dichte von flüssigem Propen beim Sätti-gungsdruck $d \frac{20}{4} = 0{,}51934$ g/ml beträgt, entspricht diese Beschickungsgeschwindigkeit etwa 6,7 bis 123,4 Mol Propen pro Liter Katalysator und Stunde. Bei dem bekannten Verfahren sollen 20 bis 90 Molprozent des eingesetzten Propens pro Durchlauf umgewandelt werden können, WObei ein Konversionsgrad von etwa 35% bevorzugt wird. Unter diesen Bedingungen wurde die beste Selektivität für Iso-propylalkohol, im folgenden mit IPA bezeichnet, bei 135°C erreicht, jedoch betrug sie nur 69 Molprozent des nur zu 22 Molprozent umgewandelten Propens.

Die Selektivität der Nebenprodukte betrug für Diisopropyläther etwa 28 und für Propenpolymerisate 3 Molprozent.

Wie diese Auslegeschrift lehrt, nimmt der relativ geringe Umwandlungsgrad des Olefins bei höherer Arbeitstemperatur zwar noch etwas zu, jedoch stieg die Polymerisatbildung gleichfalls an und die Selektivität für IPA ging noch weiter zurück. Außerdem erwiesen sich Temperaturen über etwa 149°C als nachteilig für die nutzbare Lebensdauer des Katalysators. Bei dem bekannten Verfahren war es sowohl notwendig wie schwierig, die Temperaturschwankungen in der Katalysatorschicht innerhalb eines Bereiches von etwa 11°C, vorzugsweise 5,5°C zu halten, insbesondere bei höheren Konversionsgraden, obwohl man diesen durch lokale überhitzung des Katalysatormaterials verursachten Schwierigkeiten durch ein relativ hohes Wasser/Olefin-Molverhältnis von etwa 4 bis 10 : 1 abzuhelfen versuchte.

Ein ähnliches Verfahren ist in der DE-AS 1 105 403 beschrieben, wo als Katalysator ein sulfoniertes Mischpolymerisat aus etwa 88 bis 94% Styrol und 12 bis 6% p-Divinylbenzol verwendet wurde, das 12 bis 16 Gewichtsprozent Schwefel in Form von Sulfonsäuregruppen enthielt und in dem 25 bis 75% der Protonen dieser Säuregruppen durch Metalle aus den Gruppen I oder VIII des Periodensystems, insbesondere Cu, ersetzt waren. Bei diesem Verfahren ist eine Reaktionstemperatur von 120 bis 220, insbesondere 1.55 bis 220°C, eine Beschickungsgeschwindigkeit von 0,5 bis 1,5 Raumteilen flüssiges Olefin je Raumteil Katalysator und Stunde sowie ein Wasser/Olefin-Molverhältnis von 0,3 bis 1,5 angegeben worden. Wie die Beispiele dieser Auslegeschrift zeigen, wird auch bei diesem Verfahren eine annehmbare Selektivität für IPA nur bei niederer Temperatur, etwa 120°C, und geringem Konversionsgrad, etwa 3,9 Molprozent, erreicht. Bei höherer Temperatur (170°C) stieg zwar die Propenkonversion auf etwa 35 Molprozent an, jedoch fiel dabei die IPA-Selektivität auf 55% ab, und dieser enthielt etwa 45% Diisopropyläther, Das bekannte Verfahren ist auf Grund seiner geringen Selektivität für IPA wirtschaftlich nur tragbar, wenn man den hohen Ätheranteil zulassen, d.h. einer Verwertung zuführen kann. Wie insbesondere die DE-AS 1 291 729 zeigt, haftet den bekannten Verfahren ferner der Nachteil einer relativ kurzen nutzbaren Lebensdauer des als Katalysator verwendeten stark sauren Ionenaus-tauschers an.

In der DE-PS 2 233 967 wird ein verbessertes Verfahren zur Erzeugung von IPA beschrieben, das in einem als Rieselsäule ausgebildeten Reaktor durchgeführt wird, wobei Propylen und Wasser im Gleichstrom geführt werden, Nachteil dieses Verfahrens ist ein Umwandlungsgrad des Propens von nur 75%, der eine Rückgewinnung des Propens aus dem Restgasstrom erforderlich macht, und die Selektivität, die bei nur etwa 95% liegt.

In der DE-PS 2 429 770 wird ein Verfahren beschrieben, bei dem Isopropylalkohol in einem Reaktor hergestellt wird, wobei Propen und Reaktionswasser in den Sumpf eingespeist und im Gleichstrom durch den Reaktor geführt werden. Dieses Verfahren erreicht zwar bei geringen Propenumsätzen von ca. 10% per Durchgang noch eine Selektivität von 99%, die aber bei einer Beladung der überkritischen Gasphase mit ca. 20% IPA auf unter 95% absinkt. Nachteil ist auch hier der geringere Gesamtumsatz, der eine Rekonzentrierung des ca. 85%igen Restgases erforderlich macht.

Es werden auch Verfahren zur Verbesserung der Selektivität beschrieben, die durch Rückführung des gebildeten Ethers in den Einsatzstrom die Gleichgewichtslage unterdrücken, siehe z.B. Chemical Engineering, September 4, 1972, s. 50, 51 und DE-OS 2 759 237.

Alternative Möglichkeiten zur Erhöhung der Gesamtselektivität eines kontinuierlichen Prozesses bestehen durch separate Spaltung des gebildeten Ethers in entweder Ausgangsolefin und Alkohol, siehe z.B. die US-PS 4 352 945 oder in zwei Moleküle Alkohol pro Mol Ether in Gegenwart eines Wasserüberschusses, ziehe die DE-OS 3 336 644.

Die beiden letztgenannten Möglichkeiten haben u.a. den Nachteil, daß zu ihrer Realisierung ein weiterer Reaktor benötigt wird. Bei der erstgenannten Möglichkeit, der Etherrückführung in den Eingangsstrom, ergibt sich ein starker Abfall der Reaktorleistung.

In der DE-PS 3 419 392 wird ein Verfahren beschrieben, bei dem der Ether separat von den Reaktanden 5 bis 30% vor Ende der Reaktionsstrecke, bezogen auf die Gesamtl#nge, in den Reaktor eingespeist wird. Bei diesem Verfahren muß der Ether für die Rückführung jedoch erst destillativ oder extraktiv aus dem Rohalkoholgemisch gewonnen werden.

Es sind auch bereits verschiedene Verfahren zur Herstellung von insbesondere tertiären Alkoholen durch Hydratisierung entsprechender olefinisch ungesättigter Verbindungen in Gegenwart von Kationenaustauscherharzen vom Sulfonsäuretyp bekannt. Ausreichende Ausbeuten wurden nur dadurch erzielt, daß Lösungsmittel als Lösungsvermittler zugesetzt wurden, die dafür sorgen, daß Wasser und sec-Olefin eine einheitliche Phase bilden, oder das Olefin in einer höheren Konzentration im Prozeßwasser vorliegt. Erwähnt sei z.B. ein Verfahren, bei dem Isobuten oder ein Isobuten enthaltender Kohlenwasserstoffstrom mit einer wäßrigen Lösung einer organischen Säure in Gegenwart eines sauren Kationenaustauscherharzes als Katalysator umgesetzt wird, siehe die DE-OS 2 430 470; ferner ein Verfahren, bei dem dem Reaktionssystem ein einwertiger Alkohol zugesetzt und ein Katalysator wie oben verwendet wird, siehe japanische Patentanmeldung 137906/75. Gemäß der US-PS 4 096 194 werden Glykol, Glykolether oder Glykoldiether zugesetzt, und die JP-OS 59802/76 (Chemical Abstracts, Vol. 86, 1977, 86:19392 b) offenbart die Umsetzung in Gegenwart polarer Lösungsmittel, insbesondere Dioxan.

In der DE-OS 3 029 739 werden mehrwertige Alkohole vom Neopentyltyp und in der DE-OS 2 721 206 und DE-OS 3 031 702 Sulfone, z.B. Sulfolan, als Lösungsvermittler vorgeschlagen.

Diese bekannten Verfahren zur Herstellung von tertiären Alkoholen, insbesondere tert-Butylalkohol, durch Hydratisierung von sec-Olefinen und insbesondere Isobuten besitzen den Nachteil, daß sie Nebenprodukt erzeugen, wie Additionsprodukte aus Isobuten und den zugesetzten organischen Säuren oder organischen Lösungsmitteln, obgleich diese die Reaktionsgeschwindigkeit in gewissem Ausmaß verbessern. Da diese Nebenprodukte und zugesetzten Lösungsmittel Siedepunkte in der Nähe von oder unterhalb des tert-Butylalkohols besitzen, ist ihre Abtrennung und die Isolierung des tert-Butanols von diesen Nebenprodukten und Lösungsmitteln sehr schwierig und erfordert hohe Betriebskosten. Während das Verfahren nach DE-OS 2 721 206 zur Herstellung von sec-Butanol durch Umsetzung von Buten-1 und Buten-2 bei einer Temperatur von 100 bis 220°C gute Stabilität des verwendeten Lösungsmittels zeigt, ist es unmöglich, auf diese Weise tert-Butanol in hoher Ausbeute durch selektive Hydratisierung von Isobuten zu erzeugen, da die mit anwesenden n-Olefine auch mit Wasser reagieren und neben tert-Butylalkohol auch sec-Butylalkohol und Isobuten-Dimere in größerem Umfang entstehen. Bei Verwendung organischer Säuren wie z.B. Essigsäure, müssen Maßnahmen zur Eingrenzung der Korrosionsprobleme vorgenommen werden.

Gemäß DE-AS 1 176 114 werden Verfahren vorgeschlagen, die ohne Lösungsvermittler durchgeführt werden, die aber zu geringeren Umsätzen oder zu geringeren Selektivitäten führen. In der DE-OS 3 031 702 ist aus Tabelle 2, Seite 14, ersichtlich, daß ohne Lösungsvermittler kaum wirtschaftlich brauchbare Isobutenumsätze zu erzielen sind.

Der Erfindung liegt daher die Aufgabe zugrunde, ein Verfahren bereitzustellen, das es gestattet, durch Direkthydratation von Olefinen ohne Ätherrückführung und dessen separate Spaltung Alkohole in guter Ausbeute und hoher Selektivität und insbesondere aus Isoolefinen ohne den Zusatz von Lösungsvermittlern und den damit verbundenen Nachteilen tertiäre Alkohole mit guten Ausbeuten und Selektivitäten herzustellen.

Diese Aufgabe wird erfindungsgemäß durch die Merkmale des Hauptanspruches gelöst.

Hierbei kann man entweder im Sumpfphasenverfahren den Kohlenwasserstoffstrom und das Prozeßwasser am Sumpf der jeweiligen Reaktoren oder im Rieselverfahren die beiden Prozeßströme am Kopf der jeweiligen Reaktoren zuführen. Vorzugsweise führt man das erfindungsgemäße Verfahren in einem Reaktorsystem durch, das 2 bis 10 und insbesondere 3 bis 5 Reaktoren umfaßt.

Bei gleicher Fahrweise kann das erfindungsgemäße Verfahren auch in einem einzigen Reaktor durchgeführt werden, der mehrere als separate Reaktionszonen enthält, wobei der eine Prozeßstrom von oben nach unten und der andere Prozeßstrom von unten nach oben geführt und bei Durchströmung der einzelnen Reaktionszonen im Gleichstrom die Prozeßströme beim Sumpfphasenverfahren am Sumpf der jeweiligen Zone und beim Rieselverfahren am Kopf der jeweiligen Zone eingeleitet werden. Vorteilhafterweise wird der das Reaktorsystem verlassende, im folgenden als "Restgas" bezeichnete Kohlenwasserstoffstrom zur Entfernung des Alkohols mit Wasser gewaschen und dieses dann als Prozeßwasser oder Teil des Prozeßwassers eingesetzt.

Das Verfahren der Erfindung wird unter bereits bekannten Verfahrensbedingungen durchgeführt:

Bei der Umsetzung von Propen beträgt die Temperatur 120 bis 200°C, vorzugsweise 130 bis 170°C. Der Reaktionsdruck beträgt 60 bis 200 bar, vorzugsweise 80 bis 120 bar. Das Molverhältnis von Wasser zu Propen beträgt 1 bis 50 : 1, vorzugsweise 10 bis 30 : 1. Die LHSV beträgt 0,2 bis 3 Stunden$^{-1}$, vorzugsweise 0,5 bis 1 Stunde$^{-1}$.

Bei der Umsetzung von $C_4$ bis $C_5$-Isoolefinen beträgt die Temperatur 30 bis 150°C, vorzugsweise 60 bis 110°C. Der Reaktionsdruck beträgt 10 bis 50 bar, vorzugsweise 10 bis 20 bar. Die Gesamt-LHSV beträgt 0,2 bis 5 Stunden$^{-1}$, vorzugsweise 0,2 bis 2 Stunden$^{-1}$. Das Molverhältnis von Wasser zu Isobuten beträgt 20 bis 150 : 1, vorzugsweise 40 bis 90 : 1.

Bei der erfindungsgemäßen Herstellung von Isopropylalkohol wird ohne Rekonzentrierung des Restgases in einem Durchgang ein Propenumsatz bis zu 99,5 % erreicht. Die Selektivität des Verfahrens liegt bei 99,0 bis 99,5%.

Die vorliegende Erfindung stellt somit u.a. ein Verfahren zur Herstellung von Isopropylalkohol bereit, bei dem Propen oder ein propenhaltiges Kohlenwasserstoffgemisch mit Wasser in Gegenwart eines starksauren Kationenaustauscherharzes, insbesondere eines solchen vom Sulfonsäuretyp, umgesetzt wird. Das Verfahren ist dadurch gekennzeichnet, daß der propenhaltige Kohlenwasserstoffstrom am Sumpf des ersten Reaktors und das Prozeßwasser am Sumpf des letzten Reaktors zugeführt wird. In den einzelnen Reaktoren befinden sich Prozeßwasser und Kohlenwasserstoffstrom im Gleichstrom. Bezogen auf das Gesamtsystem der Reaktoren wird jedoch der Kohlenwasserstoffstrom kaskadenartig vom ersten bis zum letzten und im Gegenstrom dazu das Prozeßwasser kaskadenartig vom letzten bis zum ersten Reaktor geführt.

Am Kopf des ersten Reaktors wird ein wäßriger Isopropylalkohol abgezogen, der in bekannter Weise destillativ aufgearbeitet wird. Je nach Reaktionsbedingungen hat der gewonnene Isopropylalkohol eine Reinheit von 99 bis 99,9%.

Er enthält nur geringe Anteile (0,1 bis 0,2%) Diisopropylether (DIPE), bezogen auf den 100%igen Alkohol. Die größere Menge des Ethers (DIPE) – ca. 0,4 bis 0,6%, bezogen auf die gebildete Alkoholmenge – wird mit dem Restgasstrom aus dem Reaktor ausgetragen.

Das Restgas enthält nur noch geringe Anteile Propen, so daß eine Rückgewinnungsoperation nicht mehr erforderlich ist.

Das Verfahren kann auch als Rieselverfahren in der erfindungsgemäßen Fahrweise betrieben werden. Dabei wird der propenhaltige Kohlenwasserstoffstrom am Kopf des ersten Reaktors eingespeist. Das Prozeßwasser wird am Kopf des letzten Reaktors eingespeist, nach Phasentrennung am Sumpf dieses Reaktors abgezogen und gegenläufig zum Kohlenwasserstoffstrom am Kopf des nächsten Reaktors erneut eingesetzt. Am Sumpf des ersten Reaktors wird schließlich der wäßrige Rohalkohol gewonnen und destillativ aufbereitet. Am Sumpf des letzten Reaktors wird ein Restgas abgezogen, das nur noch wenig Propen enthält.

Der bei der Umsetzung von $C_4$- bis $C_5$-Isoolefinen als Nebenprodukt gebildete sekundäre Alkohol kann als Seitenstrom bei der Azeotropierung des tertiären Alkohols aus der Vorentwässerungskolonne entfernt werden. Der Seitenabzug erfolgt insbesondere wenige Böden, vorzugsweise 1 bis 7 Böden, oberhalb des Einspeisbodens für den wäßrigen Alkohol.

Es wurde gefunden, daß auch ohne die Zugabe von Lösungsvermittlern hohe Isobutenumsätze und hohe Selektivitäten erreicht werden können, und zwar können beim erfindungsgemäßen Verfahren ohne jede Verwendung von Lösungsvermittlern Isobutenumsätze von über 98% erzielt werden. Die Selektivität des Verfahrens liegt bei 99 bis 99,9%. Das den letzten Reaktor verlassene Raffinat ist nach der Wasserwäsche praktisch frei von Alkohol.

Wie bei der Umsetzung mit Propen weist das Verfahren der Erfindung aufgrund seiner Fahrweise gegenüber den bekannten Verfahren auch bei der Umsetzung von $C_4$- bis $C_5$-Isoolefinen den Vorteil aus, daß auch bei Kohlenwasserstoffgemischen mit niedrigem Olefingehalt das Olefin fast vollständig umgesetzt wird.

Die vorliegende Erfindung stellt somit ein Verfahren zur Herstellung von Alkoholen, insbesondere von Isopropylalkoholen (IPA) und tert-Butylalkohol (TBA) bereit, bei welchem ein olefinhaltiger Kohlenwasserstoffstrom, vorzugsweise Propen bzw. Isobuten, oder ein diese Olefine enthaltender Kohlenwasserstoffstrom, mit Wasser in Gegenwart eines starksauren Kationenaustauscherharzes, insbesondere eines solchen vom Sulfonsäuretyps, umgesetzt wird. Das Verfahren kann darin bestehen, daß der olefinhaltige Kohlenwasserstoffstrom am Sumpf des ersten Reaktors und das Prozeßwasser am Sumpf des des letzten Reaktors zugeführt wird. In den einzelnen Reaktoren befinden sich Prozeßwasser und Kohlenwasserstoffstrom zwar im Gleichstrom, jedoch wird das Prozeßwasser vom letzten zum ersten Reaktor kaskadenartig im Gegenstrom zum Kohlenwasserstoffstrom geführt, der das Reaktorsystem vom ersten bis zum letzten Reaktor durchströmt.

Am Kopf des ersten Reaktors wird ein wäßriger Alkohol abgezogen und daraus durch Destillation der Alkohol gewonnen. Je nach Reaktionsbedingungen hat der gewonnene Alkohol eine Reinheit von 99 bis 99,9%. Bei der Umsetzung von Isobuten kann der gewonnen TBA neben geringen Mengen sec-Butylalkohol (SBA) noch geringste Mengen dimere Verbindungen enthalten. Solche Diisobutene sind im $C_4$-Restgas und nicht in dem aus der Wasserphase gewonnen TBA enthalten.

Wie bei der erfindungsgemäßen Umsetzung von Propen kann das Verfahren auch zur Umsetzung von Isoolefinen als Rieselverfahren in der erfindungsgemäßen Fahrweise betrieben werden. Dabei wird der isoolefinhaltige Kohlenwasserstoffstrom am Kopf des ersten Reaktors eingespeist. Das Prozeßwasser wird am Kopf des letzten Reaktors eingespeist, am Sumpf dieses Reaktors abgezogen und gegenläufig zum Kohlenwasserstoffstrom am Kopf des nächsten Reaktors erneut eingesetzt. Durch eine jeweilige Phasentrennung in den einzelnen Reaktoren kann die erhaltene wäßrige Phase gewonnen und in die weiteren Reaktoren eingeleitet werden. Am Sumpf des ersten Reaktors wird schließlich der wäßrige Rohalkohol gewonnen und destillativ aufbereitet. Am Sumpf des letzten Reaktors wird ein Restgas abgezogen, das nur noch wenig Isobuten enthält.

Das Verfahren der Erfindung weist bei der Umsetzung des Isoolefins aufgrund seiner Gegenstrom-Fahrweise gegenüber den bekannten Verfahren, die die Prozeßströme im Gleichstrom durch das aus einer oder mehreren Reaktionszonen bestehenden Reaktorsystem führen, neben hohen Olefinumsätzen und hoher Selektivität einen weiteren Vorteil aus. Da das Verteilungsgleichgewicht von TBA zwischen der wäßrigen Phase und der Kohlenwasserstoffphase unter den gewählten Reaktionsbedingungen etwa 1 : 3,5 beträgt, enthält das Restgas bei der Gleichstrom-Fahrweise des Standes der Technik hohe Anteile an TBA (12 bis 30%). Zur Abtrennung von TBA aus dem Restgas müßte daher eine mehrstufige Extraktion oder eine Destillation angewandt werden. Beim erfindungsgemäßen Verfahren enthält das Restgas beim Verlassen des letzten Reaktors lediglich 0,2 bis 2,5% TBA. Durch eine Nachwaschung mit dem Prozeßwasser oder einem Teil desselben (etwa in einem Gegenstromreaktor) kann das Restgas praktisch TBA-frei (> 0,1% TBA) erhalten werden. Die Abtrennung von TBA aus dem Prozeßwasser wird in einer Destillationskolonne durchgeführt, wobei am Kopf der Kolonne ein azeotroper TBA mit 12% Wasser anfällt und am Sumpf der Kolonne überschüssiges Prozeßwasser abgezogen und dem letzten Reaktor erneut zugeführt wird.

Mit dem erfindungsgemäßen Verfahren können die Nachteile der Gleichstromfahrweise des Standes der Technik Erfordernis einer hohen Olefinkonzentration Frischgas, unzureichender Olefinumsatz, unbefriedigende Selektivität, und die daraus erforderlichen Nachverarbeitungsmaßnahmen wie u.a. die destiliative Etherabtrennung, Etherspaltung, Alkoholextraktion beseitigt werden.

Die Vorteile des erfindungsgemäßen Gegenstromverfahrens können nämlich nicht nur durch Neubau oder vollständigem Ersatz einer bestehenden Gleichstromanlage genutzt werden, sondern auch durch Umbau bestehender Gleichstromreaktoren in Gegenstromreaktoren oder Ergänzung der Anlagen durch zusätzliche Gegenstromreaktoren, in welchen das olefinreiche und mit Nebenprodukten beladene Restgas der Gleichstromreaktoren so umgesetzt wird, daß auch ohne die bisherigen Nachverarbeitungsmaßnahmen ein guter Olefinumsatz von 98 bis 99% und eine gute Selektivität von 99% bei befriedigender Reaktorleistung erreicht werden.

Damit werden beim Vorliegen einer Gleichstromanlage die Vorteile des erfindungsgemäßen Verfahrens mit geringerem Investitionsaufwand erreicht.

Die Kombination von Gleichstromverfahren und Gegenstromverfahren läßt sich grundsätzlich beim Einsatz beliebiger, für die Direkthydratation von Olefinen geeigneter Feststoff-Katalysatorsysteme anwenden. Es ist auch möglich, andere Katalysatorsysteme im Gleichstromverfahren mit dem hier bevorzugten sauren Kationenaustauscherkatalysator im Gegenstromreaktor zu kombinieren.

In den Zeichnungen sind Ausführungsbeispiele der Erfindung dargestellt. Sie werden im folgenden näher beschrieben.

Es zeigen:

Fig. 1 ein Fließschema des erfindungsgemäßen Sumpfverfahrens;
Fig. 2 ein Fließschema des erfindungsgemäßen Rieselverfahrens;
Fig. 3 ein Fließschema des Rieselverfahrens im Gleichstrom gemäß dem Stand der Technik.

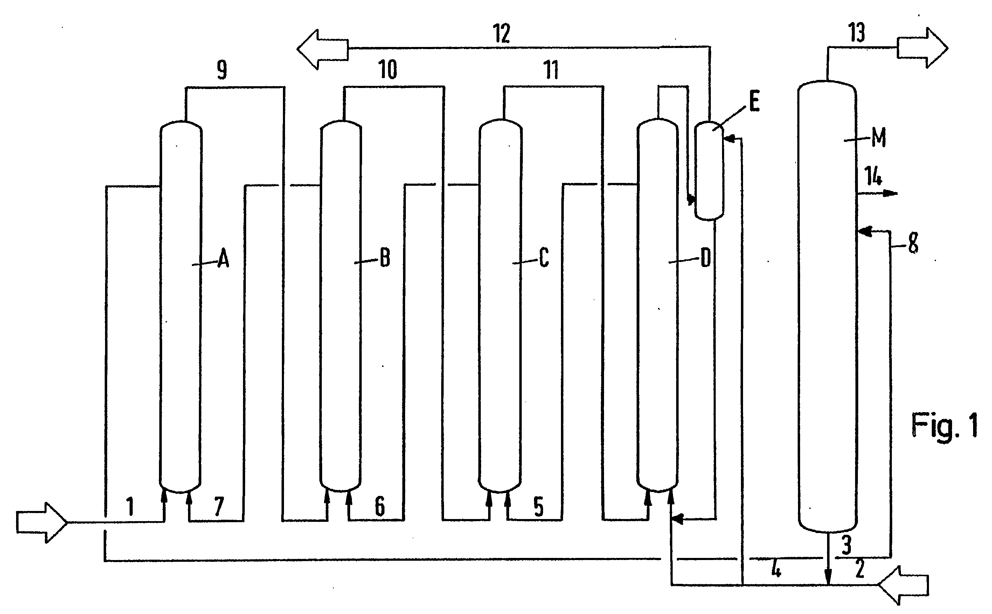

Bei einem Sumpfverfahren gemäß Fig. 1 mit einem Reaktorsystem bestehend aus 4 Reaktoren A, B, C, D wird olefinhaltiges Kohlenwasserstoff-Gemisch ($C_3$- bzw. $C_4$/$C_5$-Schnitt) über Leitung 1 am Sumpf des Reaktors A zugeführt. Prozeßwasser wird über Leitung 2 mit dem überschüssigen Wasser aus dem Sumpf der Kolonne M aus Leitung 3 vereinigt und durch Leitung 4 dem Sumpf des letzten Reaktors D zugeführt. Bei der Umsetzung der $C_4$- bis $C_5$-Olefine kann auch alles oder ein Teil des Prozeßwassers über Extraktor E geführt werden.

Die im Kopfbereich des Reaktors D über Leitung 5 abgezogene wäßrige Phase wird dem Sumpf des Reaktors C und über Leitungen 6 und 7 den jeweils vorhergehenden Reaktoren B und A und schließlich über Leitung 8 der Kolonne M zugeführt. Der am Kopf des Reaktors A über Leitung 9 abgezogene Kohlenwasserstoffstrom wird dem Sumpf des Reaktors B und dann über Leitungen 10 und 11 den jeweils folgenden Reaktoren C und D zugeführt und schließlich bei der Propen-Umsetzung über Leitung 12 als Restgas abgeführt. Bei der Umsetzung der $C_4$- bis $C_5$-Isoolefine wird das Restgas zweckmäßigerweise zuvor zur Entfernung von darin enthaltenem TBA noch im Extraktor E gewaschen. Hierfür wird das Prozeßwasser aus Leitung 4 oder ein Teilstrom des Prozeßwassers aus Leitung 4 eingesetzt, der sodann mit dem restlichen Prozeßwasser wie beschrieben dem Sumpf des Reaktors D zugeführt wird. Damit werden zwar Prozeßwasser und olefinhaltige Kohlenwasserstoffe durch die einzelnen Reaktoren im Gleichstrom, durch die Reaktorkette jedoch kaskadenartig im Gegenstrom geführt.

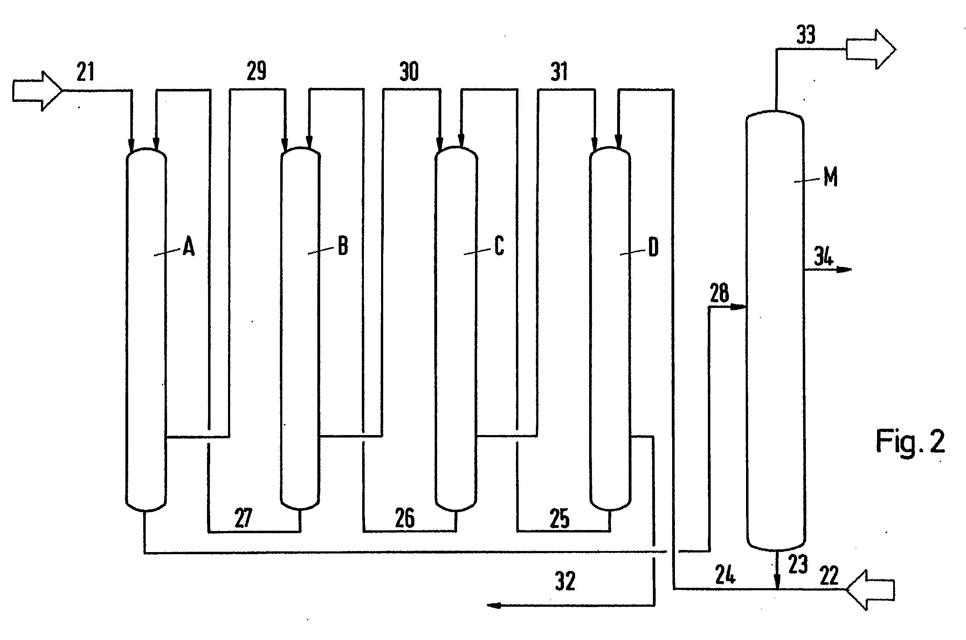

Das Verfahren der Erfindung kann auch als Rieselverfahren im Gegenstrom betrieben werden. Eine Ausführungsform ist in Fig. 2 anhand des gleichen Reaktorsystems mit 4 Reaktoren dargestellt. Bei diesem Verfahren wird olefinhaltiges Kohlenwasserstoff-Gemisch über Leitung 21 am Kopf des Reaktors A und das Prozeßwasser über Leitung 24 am Kopf des Reaktors D eingespeist und die Prozeßströme werden ebenfalls kaskadenartig im Gegenstrom durch das Reaktorsystem geführt.

Das in Reaktor D eingespeiste Prozeßwasser wird als wäßrige Phase über Leitung 25 abgezogen und dem Kopf des Reaktors C und dann über die Leitungen 26 und 27 den jeweils vorhergehenden Reaktoren B und A zugeführt. Entsprechend wird der Kohlenwasserstoffstrom in der entgegengesetzten Richtung im Sumpfbereich des Reaktors A abgezogen und über Leitungen 29, 30, 31 den folgenden Reaktoren B, C, D zugeführt und schließlich über Leitung 32 als Restgas abgeführt, das im Falle der Umsetzung der $C_4$- bis $C_5$-Isoolefine zur Entfernung von darin enthaltenem TBA noch in dem Extraktor E gewaschen wird.

Entweder im Kopfbereich (Sumpfverfahren) oder am Sumpf (Rieselverfahren) des Reaktors A wird wäßriger Alkohol über Leitung 8 bzw. Leitung 28 abgezogen und der Kolonne M zugeleitet, wobei am Kopf der Kolonne über Leitung 13 bzw. 33 azeotroper Alkohol gewonnen wird, der bei der Herstellung von TBA neben 12% Wasser 0,05 bis 1% SBA und geringste Mengen dimere Verbindungen enthalten kann. Nach Bedarf kann der azeotrope Alkohol in bekannter Weise getrocknet werden.

Gemäß einer bevorzugten Ausführungsform des Verfahrens wird in den vier hintereinander geschalteten Reaktoren die Reaktionstemperatur jeweils so erhöht, daß auch bei geringerer Wasserbelastung ein fast vollständiger Olefinumsatz (99%) und eine Selektivität von etwa 99% erreicht wird.

Bei der $C_4$-Isoolefin-Umsetzung kann die durch die Bildung von SBA verringerte Reinheit des erzeugten TBA bis zu 99,9% verbessert werden, wenn unter den Bedingungen der destillativen Aufarbeitung des wäßrigen Roh-TBA an einer geeigneten Stelle der Azeotropierungskolonne der SBA als wasserhaltiger, gegenüber TBA angereicherter Seitenstrom über Leitung 14 bzw. 34 abgezogen wird.

Das erfindungsgemäße Verfahren hat den Vorteil, ohne jegliche Rekonzentrierung des Restgases jedes beliebige Olefin enthaltende Gemisch, ob 50%ig oder 98%ig, fast quantitativ umzusetzen. Das Verfahren arbeitet selbst unter an sich ungünstigen Temperaturbedingungen (z.B. bei der IPA-Herstellungstemperaturen 150°C) aufgrund der Gegenstrom-Fahrweise sehr selektiv. Im Gegensatz zum bekannten Gleichstrom-Rieselverfahren wird mit dem Restgas z.B. bei der Propenumsetzung kaum Isopropylalkohol und Diisopropylether aus dem Reaktorsystem ausgetragen.

Die folgenden Beispiele dienen der Erläuterung der Erfindung.

Beispiele 1 bis 5

In dem in Fig. 1 dargestellten Verfahren wurden 5320 g (wasserfeucht) eines handelsüblichen starksauren Kationentauschers auf vier Reaktoren verteilt. Über Leitung 1 wurde dem ersten Reaktor (A) ein Propan/Propen-Gemisch zugeführt. Über Strom 4 wurde dem letzten Reaktor (D) Prozeßwasser zugeführt, das die Reaktoren kaskadenartig vom letzten Reaktor (D) bis zum ersten Reaktor (A) durchlief. Mengenströme, Bedingungen und Ergebnisse sind aus Tabelle I ersichtlich.

Tabelle I

| | Beispiele | | | | |
|---|---|---|---|---|---|
| | 1 | 2 | 3 | 4 | 5 |
| Temperatur, $^\circ$C | 150 | 150 | 150 | 158 | 158 |
| Druck, bar | 100 | 100 | 100 | 100 | 100 |
| Propengehalt, Mol% | 92 | 92 | 92 | 92 | 58 |
| Propangehalt, Mol% | 8 | 8 | 8 | 8 | 42 |
| Menge (g/h) Prozeß-wasser (Strom 4) | 8000 | 10000 | 10000 | 10000 | 6000 |
| Menge (g/h) Propan/Propen-Gemisch (Strom 1) | 800 | 800 | 1000 | 1000 | 1000 |
| Menge (Mol/h) Propen | 17,5 | 17,5 | 21,9 | 21,9 | 13,8 |
| Umwandlung (Mol%) | 96,7 | 99,6 | 90,8 | 99,3 | 98,3 |
| Selektivität (Mol%) für IPA | 99,5 | 99,4 | 99,0 | 99,3 | 98,9 |
| Selektivität (Mol%) für IPE | 0,5 | 0,6 | 1,0 | 0,7 | 1,1 |
| Selektivität (Mol%) für iso-Hexene plus n-Propylalkohol (NPA) | 0,1 | <0,1 | <0,1 | <0,1 | <0,1 |
| Katalysatorleistung Mol IPA/l Kat · h | 2,4 | 2,5 | 2,8 | 3,1 | 1,9 |
| DIPE im Restgas, g/h (ungewaschen) | 3,4 | 4,0 | 8,1 | 5,9 | 6,0 |

Beispiele 6 bis 8

Das Verfahren der Erfindung kann auch als Rieselverfahren betrieben werden. Eine Ausführungsform ist in Fig. 2 dargestellt.

Bei diesem Verfahren wird der Propan/Propen-Kohlenwasserstoffstrom über Leitung 21 am Kopf des ersten Reaktors (A) eingespeist und das Prozeßwasser über Leitung 24 am Kopf des letzten Reaktors (D). Der sm Sumpf des ersten Reaktors (A) abgezogene wäßrige Alkohol wird über Leitung 28 abgeführt und in bekannter Weise aufbereitet.

Die vier Reaktoren wurden mit 5320 g eines starksauren Kationenaustauschers gefüllt. Mengenströme, Bedingungen und Ergebnisse sind in Tabelle II zusammengestellt.

## Tabelle II

|  | Beispiele | | |
|---|---|---|---|
|  | 6 | 7 | 8 |
| Temperatur, $^\circ$C | 150 | 150 | 158 |
| Druck, bar | 100 | 100 | 100 |
| Propen im $C_3$-Kohlenwasserstoffgemisch, Mol% | 92 | 92 | 58 |
| Menge (g/h) Prozeßwasser (Strom 24) | 8000 | 10000 | 6000 |
| Menge (g/h) $C_3$-Kohlenwasserstoff (Strom 21) | 800 | 1000 | 1000 |
| Menge (Mol/h) Propen | 17,5 | 21,9 | 13,8 |
| Umwandlung (Mol%) | 97,2 | 92,9 | 98,5 |
| Selektivität (Mol%) zu IPA | 99,4 | 99,1 | 98,5 |
| Selektivität (Mol%) zu IPE | 0,6 | 0,9 | 1,5 |
| Selektivität (Mol%) für iso-Hexene plus n-Propylalkohol | <0,1 | <0,1 | <0,1 |
| Katalysatorleistung Mol IPA/l Kat · h | 2,4 | 2,9 | 1,9 |
| DIPE im Restgas, g/h (ungewaschen) | 4,1 | 7,4 | 8,5 |

Beispiele 9 bis 11 (Vergleichsbeispiele)

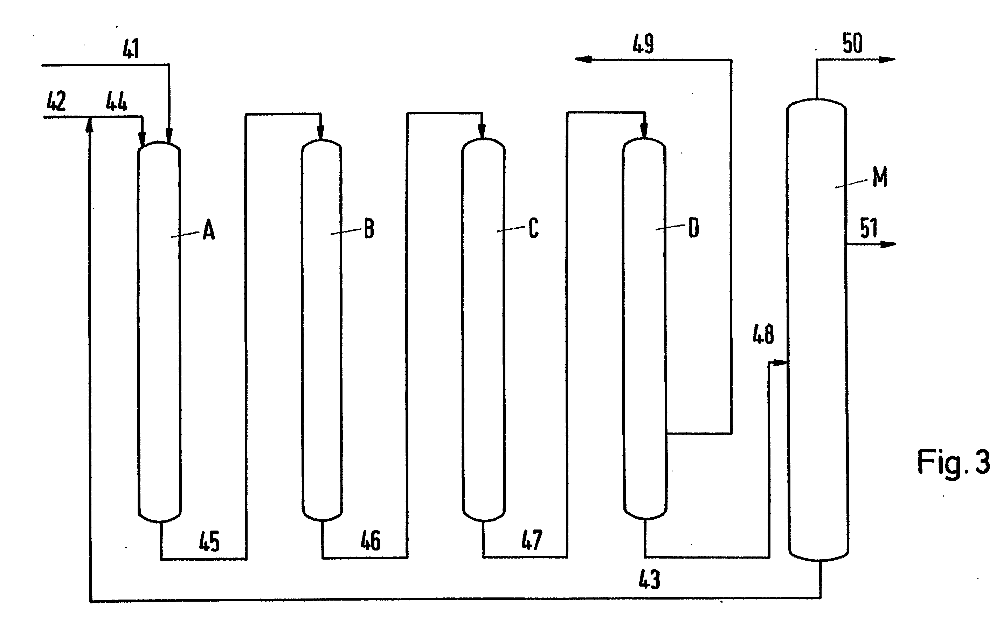

Die in Fig. 3 dargestellten Reaktoren wurden als Rieselreaktoren im Gleichstrom unter den sonst in den Beispielen 6 bis 8 beschriebenen Bedingungen betrieben.
Mengenströme, Bedingungen und Ergebnisse sind in der Tabelle III zusammengestellt.

## Tabelle III

| | Beispiele | | |
|---|---|---|---|
| | 9 | 10 | 11 |
| Temperatur, °C | 150 | 150 | 158 |
| Druck, bar | 100 | 100 | 100 |
| Propengehalt, % | 92 | 92 | 58 |
| Menge Prozeßwasser, g/h (Strom 44) | 8000 | 10000 | 6000 |
| Menge Propan/Propen, g/h (Strom 41) | 800 | 1000 | 1000 |
| Menge Propen, Mol/h | 17,5 | 21,9 | 13,8 |
| Umwandlung, Mol% | 74 | 78 | 59 |
| Selektivität (Mol%) zu IPA | 91,2 | 91,2 | 87 |
| Selektivität (Mol%) zu IPE | 8 | 8 | 12 |
| Selektivität (Mol%) für iso-Hexene plus n-Propylalkohol | 0,8 | 0,8 | 1,0 |
| Katalysatorleistung Mol IPA/l Kat · h | 1,7 | 2,2 | 1,0 |
| DIPE im Restgas, g/h (ungewaschen) | 60 | 81 | 57 |

Beispiele 12 bis 14 (Vergleichsbeispiele)

Entsprechend wurden die vier Reaktoren als Sumpfreaktoren im Gleichstrom unter den sonst in den Beispielen 6 bis 8 beschriebenen Bedingungen betrieben, Mengenströme, Bedingungen und Ergebnisse sind in Tabelle IV zusammengestellt.

## Tabelle IV

|  | Beispiele | | |
|---|---|---|---|
|  | 12 | 13 | 14 |
| Temperatur, °C | 150 | 150 | 158 |
| Druck, bar | 100 | 100 | 100 |
| Propengehalt, % | 92 | 92 | 58 |
| Menge Prozeßwasser, g/h | 8000 | 10000 | 6000 |
| Menge Propan/Propen, g/h | 800 | 1000 | 1000 |
| Menge Propen (Mol/h) | 17,5 | 21,9 | 13,8 |
| Umwandlung, Mol% | 72 | 74 | 58 |
| Selektivität (Mol%) zu IPA | 92,4 | 92,4 | 88 |
| Selektivität (Mol%) zu IPE | 7 | 7 | 11 |
| Selektivität (Mol%) zu iso-Hexenen plus NPA | 0,6 | 0,6 | 0,8 |
| Katalysatorleistung, Mol IPA/1 Kat · h | 1,7 | 2,1 | 1,0 |
| DIPE im Restgas, g/h (ungewaschen) | 51 | 66 | 52 |

Beispiel 15 bis 20

In dem in Fig. 1 dargestellten Verfahren, bei dem insgesamt 2750 g eines handelsüblichen starksauren Kationenaustauschers auf vier Reaktoren verteilt waren, wurde über Leitung 1 ein isobutenhaltiges $C_4$-Kohlenwasserstoff-Gemisch eingespeist. über Strom 4 wurde Prozeßwasser zugeführt, das die Reaktoren kaskadenartig vom letzten zum ersten durchlief. Mengenströme, Bedingungen und Ergebnisse sind aus Tabelle V ersichtlich.

## Tabelle V

### Beispiele

| | 15 | 16 | 17 | 18 | 19 | 20 |
|---|---|---|---|---|---|---|
| Temperatur, $^{o}$C | 70 | 92 | 92 | 92 | 92 | 98 |
| Druck, bar | 16 | 16 | 16 | 16 | 16 | 16 |
| Isobutengehalt, Mol% | 45 | 45 | 45 | 78,5 | 17 | 45 |
| n-Butengehalt, Mol% | 40 | 40 | 40 | 8 | 58 | 40 |
| Butane, Mol% | 15 | 15 | 15 | 3,5 | 25 | 15 |
| Menge (g/h) Prozeß-wasser (Strom 4) | 8000 | 8000 | 4000 | 4000 | 4000 | 4000 |
| Menge (g/h) Wasch-wasser | 2000 | 2000 | 2000 | 2000 | 2000 | 2000 |
| Menge (g/h) $C_4$-Kohlen-wasserstoff (Strom 1) | 600 | 600 | 600 | 600 | 600 | 600 |
| Menge Isobuten (Mol/h) | 4,8 | 4,8 | 4,8 | 8,4 | 1,8 | 4,8 |
| Umwandlung (Mol%) Isobuten | 86 | 98,5 | 92 | 96 | 90 | 98 |
| Umwandlung (Mol%) n-Butene | 0,09 | 1,0 | 0,9 | 0,3 | 1,2 | 1,3 |
| Selektivität (Mol%) für TBA | 99,9 | 99,1 | 99,1 | 99,7 | 98,8 | 98,7 |
| Selektivität (Mol%) für SBA | 0,09 | 0,9 | 0,9 | 0,3 | 1,2 | 1,3 |
| Selektivität (Mol%) dimere Verbindungen | 0,015 | 0,02 | 0,02 | 0,025 | 0,015 | 0,025 |
| TBA im Restgas, Gew.% (Strom 12) | <0,1 | <0,1 | <0,1 | <0,1 | <0,1 | <0,1 |

### Beispiele 21 bis 26

Die Beispiele 15 bis 20 wurden mit der Maßgabe wiederholt, daß jetzt die Menge des starksauren Kationenaustauschers auf drei Reaktoren aufgeteilt wurde. Bedingungen, Mengenströme und Ergebnisse sind aus Tabelle VI ersichtlich.

## Tabelle VI

|  | Beispiele | | | | | |
|---|---|---|---|---|---|---|
|  | 21 | 22 | 23 | 24 | 25 | 26 |
| Temperatur, °C | 70 | 92 | 92 | 92 | 92 | 98 |
| Druck, bar | 16 | 16 | 16 | 16 | 16 | 16 |
| Isobutengehalt, Mol% | 45 | 45 | 45 | 78,5 | 17 | 45 |
| n-Butengehalt, Mol% | 40 | 40 | 40 | 8 | 58 | 40 |
| Butane, Mol% | 15 | 15 | 15 | 3,5 | 25 | 15 |
| Menge (g/h) Prozeß- wasser (Strom 4) | 8000 | 8000 | 4000 | 4000 | 4000 | 4000 |
| Menge (g/h) Wasch- wasser | 2000 | 2000 | 2000 | 2000 | 2000 | 2000 |
| Menge (g/h) $C_4$-Kohlen- wasserstoffe (Strom 1) | 600 | 600 | 600 | 600 | 600 | 600 |
| Menge (Mol/h) Isobuten | 4,8 | 4,8 | 4,8 | 8,4 | 1,8 | 4,8 |
| Umwandlung (Mol%) Isobuten | 76 | 90 | 86 | 88 | 82 | 89 |
| Umwandlung (Mol%) n-Butene | 0,09 | 0,9 | 0,8 | 0,3 | 1,1 | 1,1 |
| Selektivität (Mol%) für TBA | 99,9 | 99,0 | 99,0 | 99,6 | 98,7 | 98,9 |
| Selektivität (Mol%) für SBA | 0,1 | 1,0 | 1,0 | 0,35 | 1,3 | 1,1 |
| Selektivität (Mol%) dimere Verbindungen | 0,015 | 0,02 | 0,02 | 0,025 | 0,02 | 0,02 |
| TBA im Restgas, Gew.% (Strom 12) | <0,1 | <0,1 | <0,1 | <0,1 | <0,1 | <0,1 |

Beispiele 27 bis 29

Die Beispiele 27 bis 29 wurden nach dem in Fig. 2 dargestellten Rieselverfahren, bei dem die vier Reaktoren mit insges. 2750 g des starksauren Kationenaustauschers gefüllt wurden, betrieben. Mengenströme, Bedingungen und Ergebnisse sind in Tabelle IV zusammengestellt.

## Tabelle VII

|  | Beispiele | | |
|---|---|---|---|
|  | 27 | 28 | 29 |
| Temperatur, °C | 70 | 92 | 98 |
| Druck, bar | 16 | 16 | 16 |
| Isobutengehalt, Mol% | 45 | 45 | 45 |
| n-Butengehalt, Mol% | 40 | 40 | 40 |
| Butane, Mol% | 15 | 15 | 15 |
| Menge (g/h) Prozeß-wasser (Strom 24) | 8000 | 8000 | 4000 |
| Menge (g/h) Wasch-wasser | 2000 | 2000 | 2000 |
| Menge (g/h) $C_4$-kohlen-wasserstoff (Strom 21) | 600 | 600 | 600 |
| Menge (Mol/h) Isobuten | 4,8 | 4,8 | 4,8 |
| Umwandlung (Mol%) Isobuten | 87,5 | 98,7 | 98,5 |
| Umwandlung (Mol%) n-Butene | 0,2 | 1,0 | 1,5 |
| Selektivität (Mol%) für TBA | 99,8 | 99,0 | 98,5 |
| Selektivität (Mol%) für SBA | 0,2 | 1,0 | 1,5 |
| Selektivität (Mol%) dimere Verbindungen | 0,03 | 0,05 | 0,05 |
| TBA im Restgas, Gew.% (Strom 32) | 0,1 | 0,1 | 0,1 |

Beispiel 30

In einem in Fig. 1 dargestellten Verfahren, bei dem die Reaktoren mit insges. 2750 g des handelsüblichen starksauren Kationenaustauschers gefüllt waren, wurden entsprechend dem Beispiel 1 über Leitung 1 600 g/h eines 45 Mol% Isobuten enthaltenden C4-Kohlenwasserstoff-Gemisches und über Leitung 48 000 g/h Prozeßwasser kaskadenartig im Gegenstrom durch die Reaktoren geführt.

Bei einer Betriebstemperatur von 70°C und einem Betriebsdruck von 16 bar wurde ein Isobutenumsatz von 86% erreicht. Demgemäß wurden über Leitung 88 232 g/h wäßriges Reaktorausgangsprodukt, das 3,72 Gew.% TBA, nur Spuren an ZBA und dimeren Produkten enthielt, abgezogen.

Dieses wäßrige Reaktionsprodukt wurde zur kontinuierlichen Destillation des TBA auf den 12. Boden einer insgesamt 50-bödigen Kolonne eingesetzt. Bei einer Einsatzmenge von 3950 g/h wäßrigem Produkt wurden als Kopfprodukt 348 g/h azeotroper TBA mit 12% Wasser und einer auf wasserfreien TBA gerechneten Reinheit von 99,9 Gew.% abgezogen. Am Sumpf der Destillationskolonne wurde bei einem eingestellten Rücklaufverhältnis von Rücklauf/Destillat = 2 ein alkoholfreies Prozeßwasser zurückgewonnen.

Beispiel 31

In einem in Fig. 1 dargestellten Verfahren, bei dem die Reaktoren mit insges. 2750 g des handelsüblichen starksauren Kationenaustauschers gefüllt war, wurden entsprechend dem Beispiel 36 über Leitung 1 600 g/h eines 45 Mol% Isobuten enthaltenden $C_4$-Kohlenwasserstoff-Gemisches und über Leitung 4 4000 g/h Prozeßwasser kaskadenartig im Gegenstrom durch die Reaktoren geführt.

Bei einer Betriebstemperatur von 98°C und einem Betriebsdruck von 16 bar wurde ein Isobutenumsatz von 98% erreicht.

Demgemäß wurden über Leitung 84 270 g/h wäßriges Reaktorausgangsprodukt, das 8,16 Gew.% TBA, 0,1 Gew.% SBA und <0,01 Gew.% Dimere enthielt, abgezogen.

Dieses wäßrige Reaktionsprodukt wurde zur kontinuierlichen Destillation des TBA auf den 12. Boden einer 70-bödigen Kolonne eingesetzt. Bei einer Einsatzmenge von 4270 g/h wäßrigem Produkt wurden am 17. Boden über Leitung 14, fünf Böden oberhalb der Produktzuführung, 14,7 g/h eines Produktstromes mit 29,2 Gew.% SBA, 49,5 Gew.% TBA und 21,3 Gew.% Wasser abgezogen. Gleichzeitig fielen am Kopf der Kolonne als Destillat 387,9 g/h mit 11,96 Gew.% Wasser und einer auf wasserfreien TBA gerechneten Reinheit von 99,9 Gew.% an.

Am Sumpf der Kolonne wurde bei einem eingestellten Rücklaufverhältnis von Rülcklauf/Destillat = 2 ein alkoholfreies Prozeßwasser zurückgewonnen.

Beispiele 32 bis 34 (Vergleichsbeispiele)

Für die Vergleichsbeispiele wurden die vier Reaktoren als Rieselverfahren im Gleichstrom betrieben (Fig. 3). Als Katalysator wurden insges. 2750 g des starksauren Kationenaustauschers eingesetzt. In diesen Versuchen wurde das isobutenhaltige $C_4$-Kohlenwasserstoff-Gemisch über Leitung 41 und das Prozeßwasser über Leitung 44! gemeinsam am Kopf des Reaktors A eingegeben, wäßriger TBA und das Raffinat am Sumpf des Reaktors D über Leitung 48 bzw. Leitung 49 abgezogen.

Reaktionsbedingungen, Mengenströme und Ergebnisse sind in Tabelle VIII zusammengefaßt.

Tabelle VIII (Vergleichsbeispiele)

| | Versuch 32 | Versuch 33 | Versuch 34 |
|---|---|---|---|
| Temperatur, °C | 70 | 92 | 98 |
| Druck, bar | 16 | 16 | 16 |
| Isobutengehalt, Mol% | 45 | 45 | 45 |
| n-Butene, Mol% | 40 | 40 | 40 |
| Butane, Mol% | 15 | 15 | 15 |
| Menge (g/h) Prozeß-wasser (Strom 44) | 8000 | 8000 | 4000 |
| Menge (g/h) Kohlen-wasserstoff (Strom 41) | 600 | 600 | 600 |
| Menge (Mol/h) Isobuten | 4,8 | 4,8 | 4,8 |
| Umwandlung (Mol%) Isobuten | 54 | 62 | 68 |
| Umwandlung (Mol%) n-Butene | 0,2 | 1,5 | 1,8 |
| Selektivität (Mol%) für TBA | 99,6 | 97,4 | 97,0 |
| Selektivität (Mol%) für SBA | 0,35 | 2,3 | 2,6 |
| Selektivität (Mol%) für dimere Verbindungen | 0,05 | 0,3 | 0,4 |
| TBA im Restgas, Gew.% (Strom 49) | 12,8 | 24,1 | 28,9 |
| SBA im Restgas, Gew.% (Strom 49) | < 0,1 | 0,35 | 0,4 |

**Patentansprüche**

1. Verfahren zur Herstellung von Isopropylalkohol und tertiären $C_4$- bis $C_5$-Alkoholen durch Umsetzung eines Kohlenwasserstoffstroms, der Propen bzw. $C_4$- bis $C_5$-Isoolefine enthält, mit Wasser in Gegenwart eines starksauren Feststoff-Katalysators bei erhöhtem Druck und erhöhter Temperatur, Abführung des Restgases und des wäßrigen Produktalkohols und Gewinnung des Alkohols dadurch gekennzeichnet, daß man den Kohlenwasserstoff am einen Ende und das Prozeßwasser am anderen Ende einer Reaktorenkette bestehend aus mehreren hintereinandergeschalteten Reaktionszonen einspeist und beide Prozeßströme durch die Reaktorenkette im Gegenstrom, jedoch durch die einzelnen Reaktoren im Gleichstrom führt.

2. Verfahren nach Anspruch 1, dadurch gekennzeichnet, daß man die Umsetzung in 2 bis 10, vorzugsweise 3 bis 5 hintereinandergeschalteten Reaktoren durchführt.

3. Verfahren nach Anspruch, oder 2, dadurch gekennzeichnet, daß man den Kohlenwasserstoffstrom und das Prozeßwasser am Sumpf der jeweiligen Reaktoren zuführt.

4. Verfahren nach Anspruch 1 oder 2, dadurch gekennzeichnet, daß man den Kohlenwasserstoffstrom und das Prozeßwasser am Kopf der jeweiligen Reaktoren zuführt.

5. Verfahren nach einem der Ansprüche 1 bis 4 zur Herstellung der tertiären Alkohole, dadurch gekennzeichnet, daß man das Restgas mit dem Prozeßwasser oder einem Teil des Prozeßwassers frei von tertiärem Alkohol wäscht.

6. Verfahren nach einem der Ansprüche 1 bis 5 zur Herstellung der tertiären Alkohole, dadurch gekennzeichnet, daß man den als Nebenprodukt gebildeten sekundären Alkohol als Seitenstrom bei der azeotropen Gewinnung des tertiären Alkohols aus der Destillationskolonne entfernt.

7. Verfahren nach Anspruch 6, dadurch gekennzeichnet, daß man den Seitenstrom wenige Böden, vorzugsweise 1 bis 7 Böden, oberhalb des Einspeisbodens für den wäßrigen Produktalkohol abzieht.

8. Verfahren nach einem der vorhergehenden Ansprüche, dadurch gekennzeichnet, daß man das Verfahren in einer Reaktorsäule mit mehreren übereinander angeordneten Reaktionszonen durchführt.

9. Verfahren nach einem der vorhergehenden Ansprüche, dadurch gekennzeichnet, daß man einer oder mehreren in an sich bekannter Weise im Gleichstromverfahren betriebenen Reaktionszonen zwei und mehr, in Gegenstromverfahren gemäß der vorhergehenden Ansprüche gefahrenen Reaktionszonen nachschaltet und das Verfahren zunächst im Gleichstrom, dann im Gegenstrom durchführt.

10. Verfahren nach einem der vorhergehenden Ansprüche, gekennzeichnet durch die Durchführung der Umsetzung in Gegenwart eines starksauren Kationenaustauscherharzes als Feststoff-Katalysator.

## Claims

1. Process for producing isopropyl alcohol and tertiary $C_4$–$C_5$ alcohols by reacting a hydrocarbon stream containing propene or $C_4$–$C_5$ isoolefins with water at elevated pressure and temperature in the presence of a strongly acidic solid catalyst, removing the residual gas and the aqueous alcohol product and producing the alcohol, characterised in that the hydrocarbon is fed at one end of a reactor chain consisting of several reaction zones connected in a tandem arrangement, the process water is fed at the other end, and the two process streams are passed in countercurrent operation through the reactor chain, but as parallel currents through each of the reactors.

2. Process according to claim 1, characterised in that the reaction is performed in 2 to 10 tandem reactors, preferably 3 to 5.

3. Process according to claim 1 or 2, characterised in that hydrocarbon stream and process water are fed at the bottom of the respective reactors.

4. Process according to claim 1 or 2, characterised in that hydrocarbon stream and process water are fed at the top of the respective reactors.

5. Process according to any of claims 1 to 4 for producing tertiary alcohols, characterised in that the residual gas is washed with the process water or part thereof in order to eliminate all tertiary alcohol.

6. Process according to any of claims 1 to 5 for producing tertiary alcohols, characterised in that the secondary alcohol formed as a by-product is removed as a side stream from the distillation column during azeotropic formation of the tertiary alcohol.

7. Process according to claim 6, characterised in that the side stream is removed several trays, preferably 1 to 7, above the feed tray for the aqueous alcohol product.

8. Process according to any preceding claim, characterised in that the process is conducted in a reaction column with several reaction zones connected in a cascade arrangement.

9. Process according to any preceding claim, characterised in that two or more reaction zones operated in a countercurrent mode according to the preceding claims are connected in series to one or more reaction zones operated in a parallel flow mode as known per se and that the process is first conducted in a parallel flow mode and then in countercurrent operation.

10. Process according to any preceding claim, characterised in that the reaction is performed in the presence of a strongly acidic cation exchange resin as a solid catalyst.

## Revendications

1. Procédé de préparation d'alcool isopropylique et d'alcools $C_4$–$C_5$ tertiaires par réaction d'un courant d'hydrocarbures renfermant du propène ou des iso-oléfines $C_4$–$C_5$ avec de l'eau en présence d'un catalyseur solide fortement acide à une pression et une température élevées, élimination du gaz résiduel et de l'alcool aqueux produit et obtention de l'alcool, caractérisé en ce qu'on introduit l'hydrocarbure à l'une extrémité de la chaîne de réacteurs constituée par plusieurs zones réactionnelles consécutives, qu'on introduit l'eau du procédé à l'autre extrémité et qu'on passe les deux courants à contre-courant à travers la chaîne de réacteurs, tandis qu'on les passe à courant parallèle à travers chacun des réacteurs.

2. Procédé selon la revendication 1 caractérisé en ce qu'on met en oeuvre la réaction dans 2 à 10 réacteurs consécutifs, de préférence 3 à 5.

3. Procédé selon la revendication 1 ou 2 caractérisé en ce qu'on introduit le courant d'hydrocarbures et l'eau du procédé à la base des réacteurs respectifs.

4. Procédé selon la revendication 1 ou 2 caractérisé en ce qu'on introduit le courant d'hydrocarbures et l'eau du procédé au sommet des réacteurs respectifs.

5. Procédé selon l'une des revendications 1 à 4 pour la préparation d'alcools tertiaires caractérisé en ce qu'on lave le gaz résiduel avec (une partie de) l'eau du procédé afin d'éliminer l'alcool tertiaire.

6. Procédé selon l'une des revendications 1 à 5 pour la préparation d'alcools tertiaires caractérisé en ce qu'on décharge le sous-produit, l'alcool secondaire, comme coupe latérale de la colonne de distillation, le déchargement étant effectué lors de la préparation azéotropique de l'alcool tertiaire.

7. Procédé selon la revendication 6 caractérisé en ce qu'on décharge la coupe latérale à plusieurs plateaux, de préférence 1 à 7, au-dessus du plateau d'alimentation d'alcool aqueux produit.

8. Procédé selon l'une des revendications précédentes caractérisé en ce qu'on met en oeuvre le procédé dans un réacteur constitué par plusieurs zones réactionnelles superposées.

9. Procédé selon l'une des revendications précédentes caractérisé en ce qu'on installe deux ou plusieurs zones réactionnelles opérées à contre-courant selon les revendications précédentes en arrière d'une ou plusieurs zone(s) réactionnelle(s) opérée(s) à courant parallèle de façon connue.

10. Procédé selon l'une des revendications précédentes caractérisé en ce qu'on met en oeuvre la réaction en présence d'une résine échangeuse fortement acide comme catalyseur solide.

Fig. 1

EP 0 257 511 B1

Fig. 2

EP 0 257 511 B1

Fig. 3

EP 0 257 511 B1